# EUROPEAN PATENT APPLICATION

(11) **EP 2 460 480 A2**
(43) Date of publication of application: **06.06.2012**
(21) Application number: 11191403.2
(22) Date of filing: 30.11.2011
(51) Int. Cl.: A61B 17/34

(54) **Thoracic port with changing elasticity**

(30) Priority: 01.12.2010 US 418562 P; 10.11.2011 US 293884
(71) Applicant: Tyco Healthcare Group LP, Mansfield, MA 02048 (US)
(72) Inventor: Haig, Fiona Middlemiss, Boston, MA Massachusetts 02116 (US); O'Prey, Cormac, Bishops Stortford, Hertfordshire CM23 4HH (GB); Ansell, Iain, Newmarket, Suffolk CB8 7DZ (GB); Cox, Michael John, Royston, Hertfordshire SG8 5QN (GB); Collier, Nicholas John, Cambridge, CB25 9JG (GB)
(74) Representative: Soames, Candida Jane

(57) **Abstract**

A surgical access assembly (100) for positioning within an opening in a tissue to provide access to a patient's body for insertion of surgical instrumentation therethrough. The surgical access assembly (100) includes a body portion (102) defining a longitudinal axis ("Y") and a passageway (112) and having a proximal portion (104) and a distal portion (108). At least a portion of the distal portion (108) is configured to transition from a more flexible, first configuration for passage into a patient's body and a more rigid, second configuration to securely maintain the distal portion (108) within the patient's body.

## Description

### BACKGROUND

This application claims priority from provisional application serial no. 61/418,562, filed December 1, 2010, the entire contents of which are incorporated herein by reference.

### 1. Technical Field

The present disclosure relates generally to devices that are configured and dimensioned for positioning within an intercostal space to facilitate access to an internal surgical work site with one or more surgical instruments. More particularly, the present disclosure relates to a thoracic port device fabricated from materials capable of changing elasticity in vivo.

### 2. Background of the Related Art

In an effort to reduce trauma and recovery time, many surgical procedures are performed through small openings in the skin, such as an incision or a natural body orifice. For example, these procedures include laparoscopic procedures and thoracic procedures, such as those that are performed to investigate, diagnose, and treat diseases of the heart and the great vessels of the thorax. Throughout the present disclosure, the term "minimally invasive" should be understood to encompass any and all such procedures.

Specific surgical instruments have been developed for use during minimally invasive surgical procedures, and typically include a shaft with an end effector, or operating portion, that is positioned at a distal end thereof. Dependent upon the requirements of the particular procedure, the surgical instrument may include, for example, graspers, clip appliers, staplers, specimen retrieval bags etc.

During a minimally invasive procedure, the clinician often creates an opening through the patient's body wall using an obturator or trocar, and thereafter, positions an access assembly, such as a cannula assembly, within the opening. The access assembly typically includes an elongate access sleeve that is configured and dimensioned to receive one or more of the above-mentioned surgical instruments such that the end effector can be positioned within an internal work site adjacent the tissue that is the subject of the procedure.

Unlike laparoscopic surgery, which requires insufflation of the abdominal cavity to provide an operative region for the clinician, thoracic surgery does not require the introduction of insufflation gas into the internal work site. Thus, the design of access assemblies intended for use during thoracic surgery can be simplified since the presence of a seal, or valve, is not essential.

In minimally invasive thoracic surgeries, the access assembly is generally inserted into a space located between adjacent ribs that is known as the intercostal space. After placement, one or more surgical instruments can be inserted into the internal work site therethrough.

In the interest of facilitating visualization, the introduction of certain surgical instruments and/or the removal of tissue specimens during minimally invasive thoracic procedures, it may be desirable to spread apart the tissue adjacent the ribs defining the intercostal space. Additionally, during these procedures, firm, reliable placement of the access assembly is also desirable in order to allow the access assembly to withstand forces that are applied during manipulation of the instrument(s) inserted therethrough. However, reducing patient trauma during introduction of the access assembly and during the surgical procedure, as well as reducing discomfort during recovery and the overall recovery time remain issues of importance.

Thoracic surgery can be an especially painful surgical procedure as intercostal nerves are located below each rib.

It would be advantageous to provide an access assembly which may be reliably placed between the ribs to reduce aggravation to the intercostal nerve.

### SUMMARY

In one aspect, the present disclosure provides a surgical access assembly according to the present disclosure is configured and dimensioned for positioning within an opening in tissue to provide access to a patient's body for insertion of surgical instrumentation therethrough. The surgical access assembly includes a body portion defining a longitudinal axis and a passageway having a proximal portion and a distal portion. At least a portion of the distal portion is configured to transition from a more flexible, first configuration for passage of the distal portion into a patient's body and a second more rigid configuration to securely maintain the distal portion within the patient's body.

In some embodiments, the body portion is configured to crosslink when transitioning from the first configuration to the second configuration.

In some embodiments, at least one chamber is disposed within the body portion of the access assembly which includes a material adapted to provide variable elasticity to the body portion upon transitioning between the first configuration and the second configuration.

A mechanism can be provided selected from the group consisting of an electric current, a magnetic field, and heat to effect the transition from the first configuration to the second configuration.

In some embodiments, an outer layer is positioned about the body portion and formed from a material that is more flexible than the body portion.

Methods of facilitating access to an internal work site beneath a patient's tissue are also provided. One method includes forming an opening in the patient's tissue and providing an access assembly including a body portion defining a longitudinal axis and a passageway and including a proximal portion and a distal portion, wherein at least a portion of the distal portion is configured to transition between a first configuration and a second configuration to vary the elasticity of the access assembly. The method includes the steps of advancing the access assembly through the opening and effecting the transition from the first configuration to the second configuration.

In some embodiments, the step of effecting the transition is selected from the group consisting of applying electric current, applying heat, applying a magnetic field, and combinations thereof.

The method may further include the step of inserting a surgical instrument through the passageway of the access assembly.

In some embodiments, the access assembly is advanced in a space between the ribs and provides access to a thoracic cavity of a patient.

### BRIEF DESCRIPTION OF THE DRAWINGS

Various exemplary embodiments of the present disclosure are described hereinbelow with reference to the drawings, wherein:

FIG. 1 is a front view illustrating a skeletal structure including a surgical access assembly positioned within the intercostal space defined between adjacent ribs in accordance with one embodiment of the present disclosure;

FIG. 2 is a bottom perspective view of the access assembly of FIG. 1;

FIG. 3 perspective cross-sectional view of an access assembly according to another embodiment of the present disclosure;

FIG. 4 is a cross-sectional view illustrating the access assembly of FIG. 3 positioned within the intercostal space; and

FIG. 5 is a side cross-sectional view of an access assembly according to yet another embodiment of the present disclosure.

### DETAILED DESCRIPTION OF THE EMBODIMENTS

Various embodiments of the presently disclosed access assembly, and methods of using the same, will now be described in detail with reference to the drawings wherein like references numerals identify similar or identical elements. In the drawings, and in the following description, the term "proximal" refers to the end of the access assembly, or component thereof, that is closer to the clinician and while the term "distal" refers to the end that is farther from the clinician.

The access port disclosed herein is described for use as a thoracic access port to provide access to the thoracic cavity. It should be understood that the access port can be utilized for access to other parts, e.g. cavities, of the patient in other minimally invasive surgical procedures.

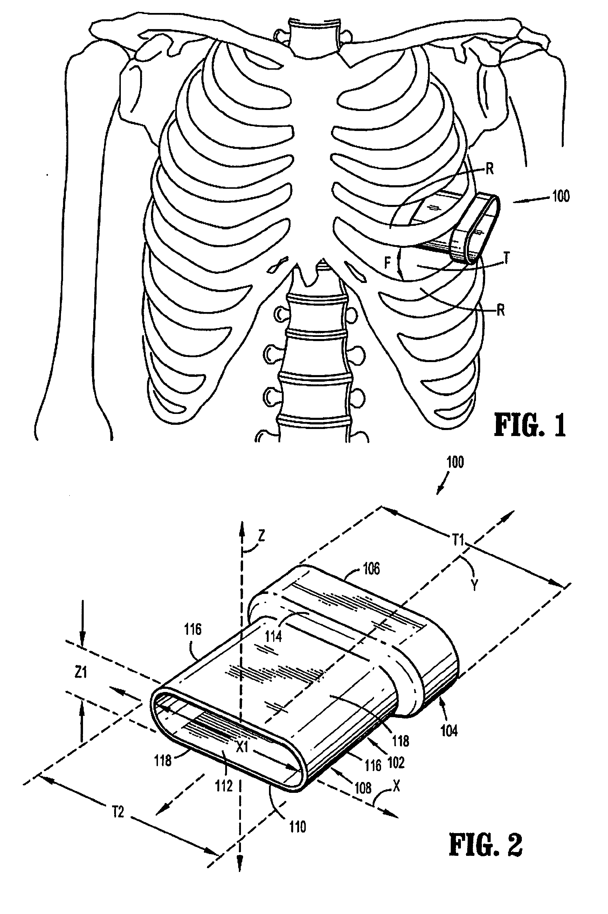

FIGS. 1-2 illustrate one embodiment of the presently disclosed surgical access assembly, which is identified by the reference character 100, for use during a minimally invasive thoracic surgical procedure. As such, the access assembly 100 is depicted as a thoracic port that is configured and dimensioned for insertion into the intercostal space located between the adjacent ribs "R" of a patient in order to allow for the insertion and manipulation of one or more surgical instruments within the thoracic cavity "T".

The access assembly 100 is configured and dimensioned to extend into the thoracic cavity "T" through the intercostal space, and includes a hollow body portion 102 that extends along a longitudinal axis "Y." The body portion 102 includes a proximal portion 104 with an open proximal end 106, a distal portion 108 with an open distal end 110, and defines an internal space 112 that is configured and dimensioned to receive one or more surgical instruments (not shown).

In one embodiment of the access assembly 100, the proximal portion 104 of the body portion 102 includes a transverse dimension "T1" that is larger than a transverse dimension "T2" defined by the distal portion 108. The larger transverse dimension "T1" of the proximal portion 104 facilitates manual engagement, e.g., gripping, by the clinician, and defines a flange, or buffer, 114 that is configured and dimensioned for abutment with the patient's tissue, e.g., the patient's ribs "R" during distal advancement of the access assembly 100 through the intercostal space. Contact between the flange 114 and the patient's tissue inhibits the access assembly 100 from passing entirely into the thoracic cavity "T" (i.e., acts as a depth stop).

In one embodiment of the access assembly 100, the distal portion 108 of the body portion 102 includes a cross-sectional configuration defining a first transverse dimension "X1" that extends along a first transverse axis "X," and a second, smaller transverse dimension "Z1" that extends along a second transverse axis "Z." The disparity between the transverse dimensions "X1," "Z1" of the distal portion 108 allows the access assembly 100 to better conform to the configuration and dimensions of the intercostal space, while maximizing available space within the body portion 102 to facilitate the insertion and manipulation of surgical instrument(s) and minimizing the application of force "F" in the direction indicated in FIG. 1, e.g., to spread the patient's ribs "R" and/or tissue adjacent the ribs "R." By minimizing the force "F," patient trauma is substantially reduced, as well as patient discomfort during recovery, and the overall recovery period.

The distal portion 108 of the body portion 102 includes a pair of opposing first sidewalls 116 and a pair of opposing second sidewalls 118. In the embodiment of the access assembly 100 illustrated in FIGS. 1-2, the pair of first sidewalls 116 is illustrated as arcuate in configuration, whereas the pair of second sidewalls 118 is illustrated as substantially planar in configuration, whereby the distal portion 108 includes an elongated, oval cross-sectional configuration. The substantially planar configuration of the pair of second sidewalls 118 maximizes the surface area available for contact with the patient's tissue.

Alternate geometrical configurations of the access assembly are within the scope of the present disclosure. For example, in an alternate embodiment of the access assembly 100, it is envisioned that both the pair of first sidewalls 116 and the pair of second sidewalls 118 may be substantially planar in configuration such that the cross-sectional configuration of the body portion 102 is substantially rectangular. Accordingly, a variety of cross-sectional configurations are contemplated for the access assembly of the present disclosure. The specific configuration and dimensions of the access assembly 100 may be varied based on such factors as the anatomy of the patient to be treated and the surgical instruments to be used in conjunction therewith.

The body portion 102 of access assembly 100 is constructed from a nondegradable, medical-grade material. In some embodiments, the access assembly 100 is fabricated from plastic and/or elastomeric materials. The access assembly 100 is capable of changing elasticity or rigidity in vivo such that it has a first configuration which enhances the ability of the clinician to introduce the access assembly into a patient's body without impinging the intercostal nerves and a second configuration which enhances the retention of the access assembly between tissues.

The material of the access assembly 100 may be capable of transforming from a relatively flexible material to a more rigid material by increasing the modulus of elasticity of the polymer of the access assembly. In some embodiments, the access assembly is fabricated from a polymer with a partially branched or crosslinked structure which is suited to further crosslink upon placement in situ or may include pre-polymers which polymerize in situ. The crosslinking reaction and/or polymerization process may include the activation of chemical moieties within the polymer and/or pre-polymers of the access assembly to form covalent bonds to create a more rigid material. Activation may occur through a variety of methods including, but not limited to, environmental changes such as pH, ionicity, and temperature; application of energy such as heat, electricity, magnetism or ultraviolet light; chemical initiated processes; etc.

In some embodiments, the access assembly 100 may be fabricated from a material containing polymerizable groups such as acrylates or methacrylates which polymerize in the presence of a UV catalyst into a crosslinked matrix. In some embodiments, an activating agent may be incorporated into the material forming the access assembly to allow the polymer to crosslink upon activation from an energy source. Activating agents include, for example photochemical groups such as alkyl azides, acyl azides, α-keto diazo compounds (a-diazo ketones, esters, etc.), diazirines, and diazoalkanes and thermochemical groups such as alkylamino, alkylcarboxyl, alkylthiol, alkylmethylimidate, alkylisocyanate, alkylisothiocyanate, alkylaldehyde, and alkylhalide, which may be react with a functionalized polymer forming the access assembly 100.

Note the access assembly can revert to the original condition when the energy, liquid, etc. is removed.

At least a portion of the distal portion 108 of the access assembly 110 should be capable of changing elasticity. In some embodiments, the entire distal portion 108 of the access assembly 110 may change elasticity, and in some embodiments, the entire or part of the distal portion 108 and the entire or part of the proximal portion 104 may change elasticity. Thus, in situ polymerization or crosslinking may occur at a location where the material is placed on, within, or both on and within, a patient.

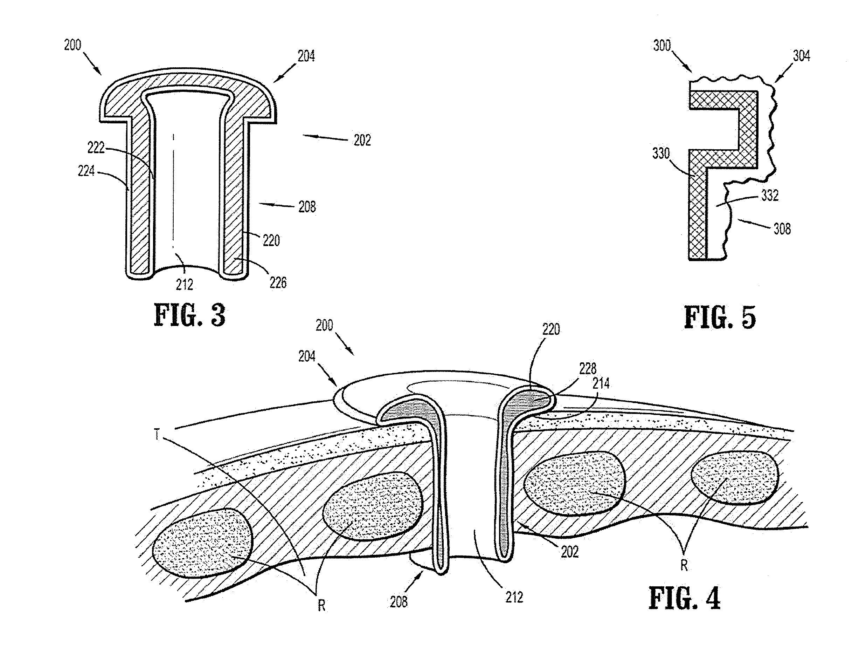

FIG. 3 illustrates an alternate access assembly 200 including at least one chamber 220 disposed within inner and outer walls 222, 224 of the body portion 202 which may filled with a fluid, gel, or a solid 226 to provide variable elasticity to the access assembly 200. The walls 222, 224 have a substantially constant modulus of elasticity which are dimensionally stable when pressure is exerted on the walls 222, 224 from within the chamber 220. Increased pressure contributes to the increased rigidity of the access assembly 200. The inner and/or outer walls 222, 224 of the body portion 202 may be a copolymer of different biocompatible materials, such as materials having different thermal characteristics, or may be a blend or mixture of two or more materials to create a polymeric material having the desired physical properties. In some embodiments, the inner and/or outer walls 222, 224 may be formed of the crosslinkable/polymerizable materials described above.

Access assembly 200 is similar to the access assembly 100 discussed above with respect to FIGS. 1 and 2, and accordingly, will only be discussed with respect to any differences therefrom. Access assembly 200 includes a body portion 202 including a proximal portion 204 and a distal portion 208. A lumen 212 is defined within the body portion 202 and is dimensioned to permit passage of one or more surgical instruments (not shown) therethrough. The body portion 202 includes at least one chamber 220 disposed within the inner wall 222 and the outer wall 224. Material 226 is disposed within the chamber that is capable of changing the elasticity, e.g., rigidity, of at least a portion of the access assembly. Thus, the access assembly has a first configuration which is pliable to ease the forces associated with insertion, placement, and/or withdrawal of the device, and a second configuration which is more rigid in order to secure the access assembly between tissue during use of the device. The more rigid configuration also aids in specimen removal through the access assembly. As noted above, after removal of the rigidifying energy or substances discussed above, the access assembly returns to its first more pliable configuration for removal. The chamber can extend the length of the body portion or be formed in only a section of the body portion. Furthermore, a plurality of chambers can be formed which can contain the same or different materials

A variable viscosity fluid may be disposed within the chamber 220 such that the viscosity of the fluid changes when heated or cooled or via application or removal of an electric current or magnetic field or through physical manipulation. In some embodiments, a ferrofluid may be disposed within the inner and outer walls 222, 224 of the access assembly 200. Typically, ferrofluids include magnetic particles, such as magnetite, dispersed and suspending in a carrier fluid and thus, tend to exhibit a change in viscosity in response to an applied magnetic field. In the presence of an electromagnetic field, the magnetic particles are induced to line up and rigidize the access assembly 200 to a degree that is proportional to the magnitude or strength of the electromagnetic field. The magnetic field may be externally applied to the access assembly 200 or alternatively, a coiled wire (not shown) may be disposed within the polymeric material forming the inner and/or outer walls 222, 224 of the access assembly 200 to generate such a magnetic field.

Polymers may also be disposed within the chamber 220 of the access assembly 200 of the present disclosure. In some embodiments, materials which are capable of adopting an alternate shape may be utilized. Shape memory polymers are generally characterized as phase segregated linear block co-polymers having a hard segment and a soft segment. The hard segment is typically crystalline, with a defined melting point, and the soft segment is typically amorphous, with a defined glass transition temperature. In some embodiments, however, the hard segment may be amorphous and have a glass transition temperature and the soft segment may be crystalline and have a melting point. The melting point or glass transition temperature of the soft segment is substantially less than the melting point or glass transition temperature of the hard segment.

When the shape memory polymer is heated above the melting point of the hard segment, the material can be shaped. This shape can be memorized by cooling the shape memory polymer below the melting point of the hard segment. When the shape memory polymer is cooled below the glass transition temperature of the soft segment, the shape may be deformed thereby forming a new temporary shape. The original shape can be recovered by heating the material above the glass transition temperature of the soft segment but below the melting point of the hard segment for removal.

Access assembly 200 may include a shape memory polymer disposed within the chamber 220 which is capable of recovering its originally memorized shape upon application of energy, such as heating, either by placement in a patient's body, or the addition of exogenous heat at a prescribed temperature, above the glass transition temperature of the soft segment but below the melting point of the hard segment of the polymer utilized. As the access assemblies of the present disclosure are utilized in a living body, heating with body heat (about 37°C) is possible.

The shape memory polymers may be compressed into a temporary shape that is smaller than its permanent shape thereby providing pliability and flexibility to the walls of the access assembly encasing the shape memory polymeric materials. The transition to its uncompressed, permanent shape increases the pressure exerted upon the walls 222, 224 to rigidize the access assembly 200. In some embodiments, the transformation from a temporary shape to a permanent shape may result in radial expansion or contraction, axial lengthening or shortening, and/or reorientation of the shape memory polymer within the chamber 220.

Similarly, in other embodiments, electrically active polymers, also known as electroactive polymers, which can alter their configuration upon application of electricity, may be utilized to fashion access assemblies in accordance with the present disclosure. Similar to the change in shape which a shape memory material may undergo upon the application of energy, such as heat, an electroactive polymer may undergo a change in shape upon the application of electricity from a low voltage electrical source (such as a battery). The application of electricity will result in the access assembly constructed of the electroactive polymer changing its shape.

While an electroactive polymer does not have the same permanent shape and temporary shape as those terms described above with respect to shape memory polymers, as used herein the term "permanent shape" as applied to an electroactive polymer means the shape the electroactive polymer adopts upon the application of electricity, and the term "temporary shape" as applied to an electroactive polymer means, the shape of the electroactive polymer adopts in the absence of electricity. In some embodiments, ferroelectric polymers may be utilized with the access assemblies of the present disclosure.

Turning now to FIG. 4, use and operation of the access assembly 200 will be discussed during the course of a minimally invasive thoracic procedure by way of example, it being understood that access assembly 200, like access assembly 100, can be used for access to other portions e.g. cavities of a patient's body. Initially, an opening is made in the patient's outer tissue wall of the thoracic body cavity "T" by conventional means, such as by puncture via an obturator (not shown). Thereafter, the access assembly 200 is inserted through the opening into the intercostal space between adjacent ribs "R". The access assembly 200 is advanced distally until the flange 214 defined by the proximal portion 204 is positioned in abutment with the patient's tissue, e.g., the patient's ribs "R."

The access assembly 200 is introduced through the opening in the patient's outer tissue wall and into the intercostal space while in the first configuration. The proximal portion 204 and/or distal portion 208 of the body portion 202 is provided in the first configuration to provide flexibility to the access assembly thereby reducing the forces applied to the patient's tissue upon placement of the access assembly 200 therein.

The body portion 202 (or portions thereof) of the access assembly 200 is configured to rigidize upon application of energy as described above. In this manner, the rigidity in the body portion 202 may result in the access assembly 200 conforming about the tissue of the intercostal space thereby restricting movement of the access assembly 200 during the course of a surgical procedure while also reducing the forces applied to the patient's tissue during the introduction and manipulation of the access assembly 200. Conformity with the specific configuration and dimensions of the intercostal space minimizes the force necessary to securely position the access assembly 200 within the tissue, which in turn, reduces patient trauma, as well as patient discomfort during recovery, and the overall recovery period.

Any surgical instrument that is configured and dimensioned to pass through the internal passageway 212 of the body portion 202 of the access assembly 200, and adapted to perform a surgical, diagnostic, or other desired procedure may be inserted through the access assembly 200 to access the surgical work site. For example, suitable surgical instruments may include endoscopic apparatus, which perform a variety of functions such as the application of surgical clips or other such fasteners to, and/or the cutting of, body tissue, specimen retrieval apparatus, graspers, etc. Following completed use of the surgical instrument, the instrument can be withdrawn from the access assembly 200 and the access assembly 200 can be removed from the intercostal space.

FIG. 5 illustrates an access assembly 300 that is a composite having a rigid inner layer 330 to resist excessive bending under conditions normally encountered during a surgical procedure and a flexible outer layer 332 to cushion against the intercostal nerves. The outer layer 332 facilitates more precise conformity with the shape of the intercostal space thereby restricting movement of the access assembly 300 during the course of the surgical procedure while maintaining a soft, pliant exterior surface to the access assembly 300. This allows for a reduction in the forces applied to the patient's tissue during the course of the surgical procedure thereby reducing the influence of such forces upon the patient's tissue and consequently patient trauma, and reducing discomfort following the procedure and recovery time.

The inner layer 330 may be formed with variable elasticity as described in any of the embodiments above. Alternatively, the inner layer may be formed from a substantially rigid material, such as a polyurethane. The outer layer 332 may be formed from a substantially compliant material, e.g., a material having a lower durometer than the material forming the inner layer 330, either partially or wholly, a material having a lower modulus of elasticity than the inner layer 330, or a material that is less rigid than the inner layer 330. Consequently, during use of the access assembly 300, the compliant outer layer 332 provides a cushioned contact area between the body portion 302 and the patient's tissue, e.g., the patient's ribs "R" and surrounding tissue (FIG. 4).

The access assembly according to the present disclosure may also be coated with a lubricant to reduce the coefficient of friction of the device as it moves over the intercostal nerves. The coating may also reduce the friction of the instrument moving over the device and the friction of the specimen being removed through the device. Lubricants include, for example, a silicone based substance (e.g., grease, gel, or the like), surgical jelly such as Surgilube_{®} by Fougera (Melville, NY), or other coatings which may be applied to facilitate insertion of an access assembly into the intercostal space. Alternatively, polymers, such as Parylene N or C, may be applied to the exterior of the access assembly to provide a lubricious finish to the access assembly.

The lubricant may include bioactive agents. The term "bioactive agent," as used herein, is used in its broadest sense and includes any substance or mixture of substances that have clinical use. Consequently, bioactive agents may or may not have pharmacological activity per se, e.g., a dye. Alternatively a bioactive agent could be any agent, which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the present access assembly in any suitable form of matter, e.g., films, powders, liquids, gels and the like.

Examples of classes of bioactive agents, which may be utilized in accordance with the present disclosure for example, include: anti-adhesives; antimicrobials; analgesics; antipyretics; anesthetics; antiepileptics; antihistamines; anti-inflammatories; cardiovascular drugs; diagnostic agents; sympathomimetics; cholinomimetics; antimuscarinics; antispasmodics; hormones; growth factors; muscle relaxants; adrenergic neuron blockers; antineoplastics; immunogenic agents; immunosuppressants; gastrointestinal drugs; diuretics; steroids; lipids; lipopolysaccharides; polysaccharides; platelet activating drugs; clotting factors; and enzymes. It is also intended that combinations of bioactive agents may be used.

Anti-adhesive agents can be used to prevent adhesions from forming between the access assembly and the surrounding tissues to which the access assembly is inserted. Some examples of these agents include, but are not limited to hydrophilic polymers such as poly(vinyl pyrrolidone), carboxymethyl cellulose, hyaluronic acid, polyethylene oxide, poly vinyl alcohols, and combinations thereof.

Other bioactive agents, which may be included as a bioactive agent include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents, such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics, such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anticonvulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins; cytotoxic drugs; chemotherapeutics, estrogens; antibacterials; antibiotics; anti-fungals; anti-virals; anticoagulants; anticonvulsants; antidepressants; antihistamines; and immunological agents.

Other examples of suitable bioactive agents, which may be included in the lubricant coating include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins, such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids, such as antisense molecules, DNA, RNA, RNAi; oligonucleotides; polynucleotides; and ribozymes.

Persons skilled in the art will understand that the devices and methods specifically described herein and illustrated in the accompanying figures are non-limiting exemplary embodiments, and that the description, disclosure, and figures should be construed merely exemplary of particular embodiments. It is to be understood, therefore, that the present disclosure is not limited to the precise embodiments described, and that various other changes and modifications may be effected by one skilled in the art without departing from the scope or spirit of the disclosure. Additionally, it is envisioned that the elements and features illustrated or described in connection with one exemplary embodiment may be combined with the elements and features of another without departing from the scope of the present disclosure, and that such modifications and variations are also intended to be included within the scope of the present disclosure. Accordingly, the subject matter of the present disclosure is not to be limited by what has been particularly shown and described, except as indicated by the appended claims.

The invention may be described by reference to the following numbered paragraphs:-
1. A surgical access assembly configured and dimensioned for positioning within an opening in tissue to provide access to a patient's body for insertion of surgical instrumentation therethrough, the surgical access assembly comprising a body portion defining a longitudinal axis and a passageway and including a proximal portion and a distal portion, wherein at least a portion of the distal portion is configured to transition from a more flexible, first configuration for passage into the patient's body and a more rigid, second configuration to securely maintain the distal portion within the patient's body.
2. The surgical access assembly of paragraph 1, wherein the body portion is configured to crosslink when transitioning from the first configuration to the second configuration.
3. The surgical access assembly of paragraph 1, including at least one chamber disposed within the body portion of the access assembly, the at least one chamber including a material adapted to provide variable elasticity to the body portion upon transitioning between the first configuration and the second configuration.
4. The surgical access assembly of paragraph 3, wherein the material disposed within the at least one chamber is a variable viscosity fluid.
5. The surgical access assembly of paragraph 4, wherein the variable viscosity fluid is a ferrofluid.
6. The surgical access assembly of paragraph 3, wherein the material disposed within the at least one chamber is a solid polymer.
7. The surgical access assembly of paragraph 6, wherein the solid polymer is a shape memory polymer.
8. The surgical access assembly of paragraph 6, wherein the solid polymer is an electroactive polymer.
9. The surgical access assembly of paragraph 1, further comprising a mechanism selected from the group consisting of an electric current, a magnetic field, and heat to effect the transition from the first configuration to the second configuration.
10. The surgical access assembly of paragraph 1, further comprising an outer layer positioned about the body portion, the outer layer being formed from a material that is more flexible than the body portion.
11. The surgical access assembly of paragraph 1, wherein the body portion is coated with a lubricant.
12. The surgical access assembly of paragraph 11, wherein the lubricant contains a bioactive agent.
13. A method of facilitating access to an internal work site beneath a patient's tissue comprising the steps of:
   forming an opening in the patient's tissue;
   providing an access assembly including a body portion defining a longitudinal axis and a passageway and including a proximal portion and a distal portion, wherein at least a portion of the distal portion is configured to transition between a first configuration and a second configuration to vary the elasticity of the access assembly;
   advancing the access assembly through the opening; and
   effecting the transition from the first configuration to the second configuration.
14. The method of paragraph 13, wherein the step of effecting the transition is selected from the group consisting of applying electric current, applying heat, applying a magnetic field, and combinations thereof.
15. The method of paragraph 13, wherein the access assembly is advanced in a space between the ribs and provides access to a thoracic cavity of a patient.
16. The method of paragraph 13, further comprising the step of inserting a surgical instrument through the passageway of the access assembly.

## Claims

1. A surgical access assembly configured and dimensioned for positioning within an opening in tissue to provide access to a patient's body for insertion of surgical instrumentation therethrough, the surgical access assembly comprising a body portion defining a longitudinal axis and a passageway and including a proximal portion and a distal portion, wherein at least a portion of the distal portion is configured to transition from a more flexible, first configuration for passage into the patient's body and a more rigid, second configuration to securely maintain the distal portion within the patient's body.

2. The surgical access assembly of claim 1, wherein the body portion is configured to crosslink when transitioning from the first configuration to the second configuration.

3. The surgical access assembly of claim 1 or claim 2, including at least one chamber disposed within the body portion of the access assembly, the at least one chamber including a material adapted to provide variable elasticity to the body portion upon transitioning between the first configuration and the second configuration.

4. The surgical access assembly of claim 3, wherein the material disposed within the at least one chamber is a variable viscosity fluid.

5. The surgical access assembly of claim 4, wherein the variable viscosity fluid is a ferrofluid.

6. The surgical access assembly of claim 3, wherein the material disposed within the at least one chamber is a solid polymer.

7. The surgical access assembly of claim 6, wherein the solid polymer is a shape memory polymer.

8. The surgical access assembly of claim 6, wherein the solid polymer is an electroactive polymer.

9. The surgical access assembly of any preceding claim, further comprising a mechanism selected from the group consisting of an electric current, a magnetic field, and heat to effect the transition from the first configuration to the second configuration.

10. The surgical access assembly of any preceding claim, further comprising an outer layer positioned about the body portion, the outer layer being formed from a material that is more flexible than the body portion.

11. The surgical access assembly of any preceding claim, wherein the body portion is coated with a lubricant.

12. The surgical access assembly of claim 11, wherein the lubricant contains a bioactive agent.

13. A method comprising the steps of:
providing an access assembly including a body portion defining a longitudinal axis and a passageway and including a proximal portion and a distal portion, wherein at least a portion of the distal portion is configured to transition between a first configuration and a second configuration to vary the elasticity of the access assembly; and
effecting the transition from the first configuration to the second configuration.

14. The method of claim 13, wherein the step of effecting the transition is selected from the group consisting of applying electric current, applying heat, applying a magnetic field, and combinations thereof.

15. The method of claim 13 or claim 14, further comprising the step of inserting a surgical instrument through the passageway of the access assembly.
